# EUROPÄISCHE PATENTSCHRIFT

(11) **EP 1 345 885 B1**
(45) Veröffentlichungstag und Bekanntmachung des Hinweises auf die Patenterteilung: **14.09.2005**
(21) Anmeldenummer: 01270517.4
(22) Anmeldetag: 28.11.2001
(51) Int. Cl.: C07C 67/00, C07C 67/12, C07C 69/618, C07C 51/567, C07C 57/42, C07C 57/44, C07C 51/353

(54) **VERFAHREN ZUR HERSTELLUNG VON ZIMTSÄUREESTER**
METHOD FOR PRODUCING CINNAMIC ACID ESTERS
PROCEDE POUR PRODUIRE DES ESTERS D'ACIDE CINNAMIQUE

(30) Priorität: 11.12.2000 DE 10061539
(43) Veröffentlichungstag der Anmeldung: 24.09.2003
(73) Patentinhaber: Symrise GmbH & Co. KG, 37603 Holzminden (DE)
(72) Erfinder: KUHN, Walter, 37603 Holzminden (DE); RICHTER, Norbert, 37688 Beverungen (DE); WALTHER, Lutz, 37603 Holzminden (DE)
(74) Vertreter: Eisenführ, Speiser & Partner
(86) Internationale Anmeldenummer: PCT/EP2001/014050
(87) Internationale Veröffentlichungsnummer: WO 2002/048086

(56) Entgegenhaltungen:
- EP-A- 0 166 283
- DE-A- 3 144 261

## Beschreibung

Die vorliegende Erfindung betrifft ein Verfahren zur Synthese von Zimtsäureestern bzw. substituierten Zimtsäureestern durch Kondensation von Aldehyd(en) mit Carbonsäureanhydrid(en) katalysiert durch Eisen(III)salze.

Der direkte Weg zu Zimtsäureestern ist durch die Kondensation von aromatischen Aldehyden mit Essigsäureestern (Claisen-Schmidt-Reaktion; (Ber. 23, 976 (1890)) möglich und kann in Gegenwart von metallischem Natrium durchgeführt werden. In Ullmanns Encyclopedia of Industrial Chemistry 6^{th} Edition, Electronic Release, 1999, Vol. A 24, 231-239 und DE 3422412 wird die Kondensation von Benzaldehyd mit Essigsäureester unter Alkoholat-Katalysator in Alkohol beschrieben.

In den genannten Fällen wird der Katalysator mindesten äquimolar zum Benzaldehyd eingesetzt und verteuert damit die Herstellkosten des Zimtsäureesters signifikant. Die Handhabung des Katalysators ist mit einem hohen Gefährdungspotenzial verbunden.

Die Perkin Reaktion (Perkin, J. Chem. Soc. 31,388 (1877)) bietet ebenfalls die Möglichkeit Zimtsäure und Zimtsäureester herzustellen, indem ein aromatischer Aldehyd wie Benzaldehyd und Essigsäureanhydrid unter Natriumacetat Katalyse umgesetzt werden. Im Houben Weyl, Methoden der organischen Chemie, 4. Aufl., Bd. VIII/3, S. 443, Georg Thieme Verlag Stuttgart, 1952 ist die Umsetzung von Benzaldehyd mit Essigsäureanhydrid und Natriumacetat bei einem Molverhältnis von 1:1.56:065 unter Rückfluss/16 h beschrieben. Nach wässriger Aufarbeitung wird Zimtsäure in 57 % Ausbeute erhalten.

Beim 24stündigen Erhitzen bzw. durch Zugabe von Pyridin kann die Ausbeute auf 70-75 % gesteigert werden.

In JP A 76/15,026; CA 1976, 85:14285h ist die Umsetzung von Benzaldehyd mit Essigsäureanhydrid und Natriumacetat bei einem Molverhältnis von 1:0.5:05 und 160°C Reaktionstemperatur/1h beschrieben. Die Ausbeute an Zimtsäure beträgt unter diesen Bedingungen 30 %. Wird Natriumacetat durch Kaliumphosphat bzw. Natriumphosphat ersetzt, steigt die Ausbeute auf 46 %.

In J.R. Johnson, Org. Reactions I. 210 (1942) wird die Umsetzung von 0.2 Mol Benzaldehyd und 0.3 Mol Essigsäureanhydrid mittels 0.12 Mol Kaliumacetat beschrieben. Das Gemisch wird bei 170°C unter Rückfluss 5 Stunden erhitzt. Das Reaktionsgemisch wird auf 1200 ml Wasser gegossen. Nicht umgesetzter Benzaldehyd wird durch Wasserdampfdestillation gewonnen. Es wird Aktivkohle zugegeben und erhitzt. Nach Filtration wird heiß 12-14 ml konz. Salzsäure zugegeben und abgekühlt. Die ausgefallenen Kristalle werden abgesaugt und getrocknet. Es werden 16-18 g Zimtsäure erhalten; die Ausbeute beträgt 55-60 % bezogen auf Einsatzmenge Benzaldehyd.

In P. Kalnin, Helv. Chim. Acta 11, 977 (1928) sind verschiedene Salze als Katalysatoren beschrieben. Es werden 1 Mol Benzaldehyd und 1.5 Mol Essigsäureanhydrid bei 180°C 8 Stunden mit 0.65 Mol Katalysator unter Rückfluss erhitzt. Die Ausbeuten an Zimtsäure betragen nach Aufarbeitung für Kaliumacetat 72 %, für Kaliumcarbonat 59 %, für Natriumcarbonat 40 %, für Natriumacetat 39 %, für Natriumphosphat 36 %, für Kaliumphosphat 20 % und für Kaliumsulfid 32 %. Neben Kalium- und Natriumsalzen sind auch Amine als Katalysatoren möglich. Für 0.33 Mol Triethylamin liegt die Ausbeute bei 19 %.

Nachteilig bei den genannten Verfahrensvarianten ist die große Katalysator-Einsatzmenge, die das Produkt verteuert und Reaktion als auch Aufarbeitung erschwert.

Aufgabe der vorliegenden Erfindung ist es daher, einen preiswerten Katalysator zu finden, der in geringen Mengen es ermöglicht, durch Kondensation von Aldehyd(en) mit Carbonsäureanhydrid(en) in Gegenwart eines Katalysators, Zimtsäureester oder Zimtsäure herzustellen.

Es wurde ein Verfahren zur Herstellung von Zimtsäureestern und/oder Zimtsäure gefunden, mit folgenden Schritten:
- Kondensation von Benzaldehyd oder substituiertem Benzaldehyd mit einem Carbonsäurenanhydrid unter Bildung des entsprechenden Anhydrids, katalysiert durch ein Eisen(III)salz ausgewählt aus der Gruppe bestehend aus Eisenacetat, Eisensubcarbonat, Eisencarbonat, Eisenoxid, Eisenoxidhydrat, Eisenoxalat, Eisenlactat, Eisentartrat und Gemische dieser Eisen(III)salze,
- Umsetzung des gebildeten Anhydrids
   (a) in Gegenwart von Alkoholen zu Zimtsäureester
      oder
   (b) mit Wasser zu Zimtsäure.

Zimtsäureester und/oder Zimtsäure für das erfindungsgemäße Verfahren sind im allgemeinen Verbindungen der Formel (I) 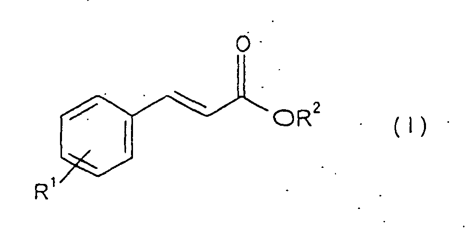 in der
- R¹: unabhängig voneinander für Wasserstoff, Alkyl, Cycloalkyl, Aryl, Aralkyl, Dialkyamino, Diarylamino, Halogen, Alkoxy, Aryloxy, Cycloalkoxy, Aralkoxy, Alkoxycarbonyl, Aryloxycarbonyl, Alkylcarbonyloxy, Alkylthio, Arylthio, Cycloalkylthio oder Aralkylthio stehen,
Alkylreste, auch in den sauerstoffhaltigen bzw. schwefelhaltigen Substituenten, sind, mit 1-16, bevorzugt 1-10,besonders bevorzugt 1-4 C-Atomen wie Methyl, Ethyl, Propyl, Isopropyl, Butyl, Isobutyl,
Cycloalkylreste, auch in den sauerstoffhaltigen bzw. schwefelhaltigen Substituenten, sind solche mit 4-8, bevorzugt 5-6, im Ring angeordneten C-Atomen, die gegebenenfalls ein oder zwei Methyl- oder Ethylgruppen tragen können, wie Cyclobutyl, Cyclopentyl, Cyclohexyl, Cycloheptyl, Cyclooctyl, Methylcyclopentyl oder Methylcyclohexyl,
als Arylreste, auch in den sauerstoffhaltigen bzw. schwefelhaltigen Substituenten, seien Phenyl, Naphthyl, Anthryl oder Diphenyl, bevorzugt Phenyl, zu verstehen, in
der R¹ für Wasserstoff, Methyl, Ethyl, Methoxy, Ethoxy oder Phenoxy steht
und
- R²: Wasserstoff oder C₁ - C₈ - Alkyl bedeuten.

Die Eisen(III)salze, die verwendet werden können sind insbesondere Eisenacetat, Eisensubcarbonat. Eisenoxid, Eisenoxidhydrat, Eisenoxalat, Eisenlactat oder Eisentartrat.

Erfindungsgemäß werden Benzoldehyd oder substituiertes Benzoldehyd eingesetzt. Beispiele sind z.B. Benzaldehyd und substituierte Benzaldehyde der Formel II, 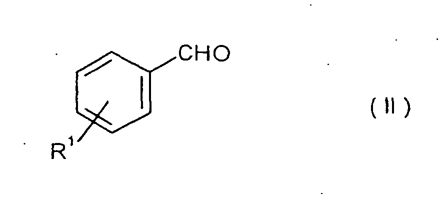 in der R¹ die oben angegebene Bedeutung hat.

Als Carbonsäureanhydride kommen C₂-C₄-Carbonsäureanhydride in Betracht. Beispiele für einsetzbare Anhydride sind Essigsäureanhydrid, Propionsäureanhydrid und Buttersäureanhydrid.

Die erfindungsgemäße Reaktion wird im allgemeinen durchgeführt, indem die Komponenten aromatischer Aldehyd, Carbonsäureanhydrid und Katalysator erhitzt werden und ein Carbonsäure-Carbonsäureanhydrid-Gemisch gleichzeitig abdestilliert wird.

Carbonsäureanhydrid wird in einer Menge von 1-5 Mol des aromatischen Aldehyds, bevorzugt in einer Menge von 1.5-2.5 Mol, eingesetzt. Überschüssiger aromatischer Aldehyd kann nach beendeter Reaktion durch Destillation zurückgewonnen und für einen neuen Ansatz verwendet werden.

Die Reaktion wird bei einer Temperatur von 100°C bis 200°C, bevorzugt bei 120°C bis 170°C durchgeführt. Das erfindungsgemäße Verfahren kann bei Normaldruck ausgeführt werden. Vorteilhafterweise wird die Reaktion unter einem Schutzgas, beispielweise unter Stickstoff durchgeführt, um Wasser (Luftfeuchtigkeit), Kohlendioxid und Sauerstoff auszuschließen.

Bei Einsatz von Benzaldehyden und Essigsäureanhydrid kann somit z.B. folgendes Gemisch erhalten werden:

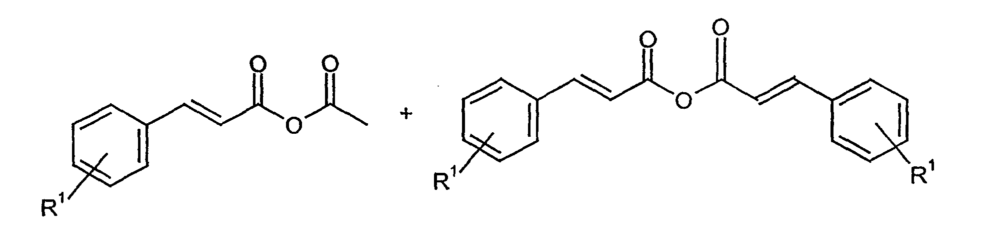

Das entstandene Anhydrid kann in Gegenwart von Alkoholen zu Estern umgesetzt werden. Vorzugsweise werden Alkohole der Formel (II) eingesetzt:

R² - OH (II)

wobei R² Wasserstoff oder C₁ - C₈- Alkyl ist.

Der Alkohol kann in einer Menge von 1-25 Mol pro Mol Benzaldehyd, bevorzugt 1.5-5 Mol eingesetzt werden

Bei Einsatz von Benzaldehyd und Essigsäureanhydrid kann demgemäß z. B. folgende Reaktion durchgeführt werden:

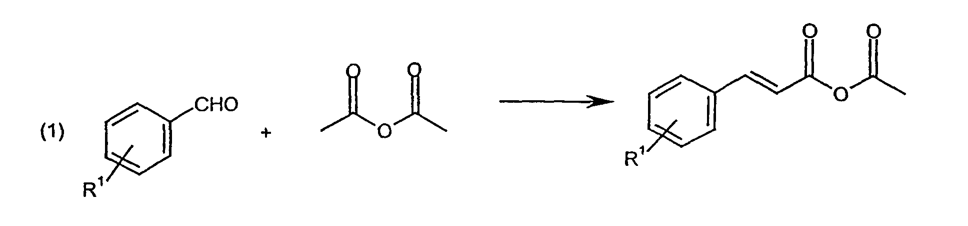

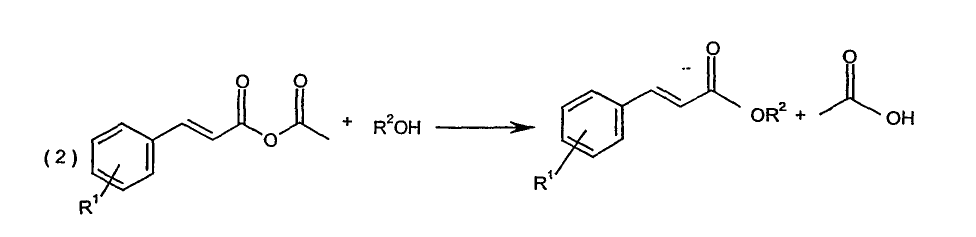

R¹ und R² haben hierbei die bereits oben angegebene Bedeutung.

Zimtsäurealkylester finden insbesondere in der Parfüm- und Essenzenindustrie Verwendung (Ullmanns Encyclopädie der technischen Chemie, 4. Auflage, Band 24, Seite 592 (1983)).

Das gebildete Anhydrid kann auch in Gegenwart von Wasser umgesetzt und Zimtsäure hergestellt werden.

Alternativ kann aber auch aus dem gebildeten Ester die Säure hergestellt werden. Dies geschieht vorzugsweise durch Verseifung. Die Verseifung kann entweder sauer oder alkalisch durchgeführt werden. Auf diese Weise kann Zimtsäure aus den gemäß obigen Reaktionsschemen gebildeten Zimtsäureestern erzeugt werden.

Die Verseifung des Esters erfolgt nach üblichen Methoden (Houben-Weyl, Band VIII, Seite 418) und wird bevorzugt alkalisch durchgeführt. Als Basen können beispielsweise wässrige Natronlauge oder wässrige Kalilauge, bevorzugt wässrige Kalilauge, eingesetzt werden. Die Base wird in einer Menge von 1-5 Mol, bevorzugt 1.5-25 Mol pro Mol der im Estergemisch vorhandenen Ester eingesetzt. Die Verseifung wird bei einer Temperatur von 20-150°C, bevorzugt 60-120°C, besonders bevorzugt bei 90-110°C durchgeführt. Aus dem Verseifungsansatz wird die freie Zimtsäure beispielsweise durch Ansäuern isoliert.

Zimtsäure und substituierte Zimtsäuren finden vielfältige Anwendung, beispielsweise zur Herstellung von Estern für die Parfüm- und Essenzenindustrie. Natriumcinnamat ist weiterhin ein Korrosionsschutzmittel (Ullmann, Band 24, Seite 592 (1983)). Zimtsäure ist weiterhin ein Zwischenprodukt für die Herstellung von Phenylalanin (JP 81/26197), zitiert nach Chem. Abstr. 95, 95470 b.

### Beispiele:

### Beispiel 1:

### Herstellung von Zimtsäuremethylester (Methylcinnamat)

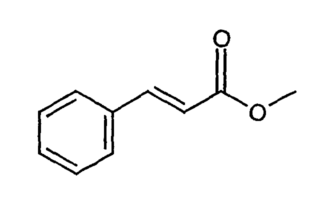

Ein Gemisch aus 1696 g (16 Mol) Benzaldehyd, 2448 g (24 Mol) Essigsäureanhydrid und 36 g (0,2 Mol) Eisensubcarbonat werden unter Rückfluss bei 145°C 0,5 Stunden erhitzt. Die Kopftemperatur fällt dabei auf 120°C. Es wird über 13 Stunden bei Normaldruck und bei einer Sumpftemperatur von 120°C bis 170°C abdestilliert. Anschließend werden 816 g (8 Mol) Essigsäureanhydrid zugegeben und nochmals über 6 Stunden bei einer Sumpftemperatur von 150°C bis 170°C abdestilliert. Es wird auf eine Sumpftemperatur von 150°C abgekühlt und bei einem Vakuum bis 50 mbar abdestilliert. Insgesamt werden 2589g eines Essigsäure-Essigsäureanhydrid-Gemisches abdestilliert.

Es werden 1200 g Toluol zugegeben und anschließend wird bei 30°C bis 78°C Sumpftemperatur ein Gemisch aus 822 g Methanol (26 Mol) und 200 g (2 Mol) konz. Schwefelsäure dosiert. Es wird 10 Stunden unter Rückfluss erhitzt.

Zum Rohansatz werden 1000 g Wasser gegeben und die Phasen getrennt. Die organische Phase wird mit 600 ml gesättigter Natriumcarbonat Lösung gewaschen. Die gewaschene organische Phase wird über eine 14bödige Kolonne im Vakuum destilliert. Bei 60°C bis 63°C/ 10 mbar gehen 208 g Benzaldehyd über. 1778 g Zimtsäuremethylester (GC-Reinheit 97.8 %) werden bei 91°C/1 mbar gewonnen. Die Ausbeute der Theorie bezogen auf Einsatz Benzaldehyd liegt bei 68 %.

### Beispiel 2:

### Herstellung von Zimtsäureethylester (Ethylcinnamat)

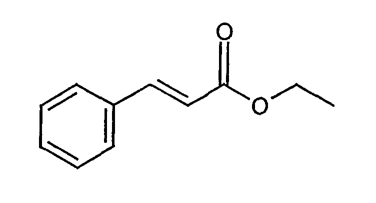

473 g (4.46 Mol) Benzaldehyd und 10 g (0.05 Mol) Eisensubcarbonat werden bei 165°C vorgelegt. Es werden 798 g (7.82 Mol) Essigsäureanhydrid bei 160 - 165°C in einem Zeitraum von 20 Stunden zudosiert und gleichzeitig bei einer Kopftemperatur von 122°C abdestilliert. Nach Dosierende wird auf 165°C aufgeheizt und bei einem Vakuum bis 50 mbar abdestilliert. Insgesamt werden 607 g eines Essigsäure-Essigsäureanhydrid-Gemisches abdestilliert.

Anschließend wird bei 130°C Sumpftemperatur ein Gemisch aus 340 g Ethanol (7.39 Mol) und 56 g (0.54 Mol) konz. Schwefelsäure dosiert. Nach Dosierende werden bei 85 - 87°C unter anziehendem Vakuum bis 500 mbar 2074 g Ethylacetat-Ethanol-Gemisch abdestilliert.

Zum Rohansatz werden 258 g 10 %ige NaOH-Lösung gegeben und die Phasen getrennt. Die organische Phase wird mit 3 g Essigsäure versetzt und über eine 14bödige Kolonne im Vakuum destilliert.

Bei 75°C bis 101°C/ 20 mbar gehen 570 g Benzaldehyd über. 424 g Zimtsäureethylester (GC-Reinheit 99.5 %) werden bei 117 - 122°C/1 mbar gewonnen.

Die Ausbeute der Theorie bezogen auf Einsatz Benzaldehyd liegt bei 54 %.

### Beispiel 3:

### Herstellung von 4-Methyl-Zimtsäuremethylester (4-Methylmethylcinnamat)

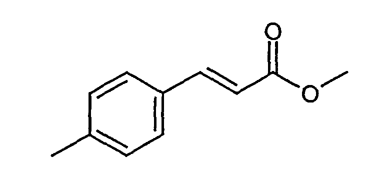

Ein Gemisch aus 1070 g (8.9 Mol) 4-Methylbenzaldehyd und 20 g (0.1 Mol) Eisensubcarbonat wird auf 165°C erhitzt. Es werden innerhalb von.24 Stunden 2040 g (20 Mol) Essigsäureanhydrid bei einer Sumpftemperatur von 165°C bis 170°C zudosiert. Gleichzeitig wird bei einer Kopftemperatur von 118°C bis 123°C ein Gemisch aus Essigsäure/Essigsäureanhydrid abdestilliert. Anschließend wird Vakuum bis 50 mbar angelegt. Insgesamt destillieren 1730 g Essigsäure/Essigsäureanhydrid-Gemisch ab. Bei fallender Sumpftemperatur von 130°C bis 75°C wird innerhalb von 2 Stunden ein Gemisch aus 1140 g Methanol (35.6 Mol) und 220 g (2.1 Mol) konz. Schwefelsäure dosiert und gleichzeitig ein Methylacetat-Methanol-Gemisch abdestilliert. Nach Dosierende wird Vakuum bis 500 mbar angelegt. Es werden insgesamt 420 g Methylacetat-Methanol-Gemisch abdestilliert.

Der Rohansatz wird bei 50°C mit 480 g 10 %iger NaOH-Lösung gewaschen. Das Rohprodukt (1452 g) wird zur Destillation mit 8 g Essigsäure versetzt und im Vakuum über eine 15 bödige Kolonne destilliert.

Bei 111°C/4 mbar gehen 100 g 4-Methylbenzaldehyd über. 820 g 4-Methyl-Zimtsäuremethylester (GC-Reinheit 98 %) werden bei 110°C/ 1 mbar gewonnen. Die Ausbeute der Theorie bezogen auf Einsatz 4-Methylbenzaldehyd liegt bei 52 %.

## Patentansprüche

1. Verfahren zur Herstellung von Zimtsäureestern und/oder Zimtsäure mit folgenden Schritten:
- Kondensation von Benzaldehyd oder substituiertem Benzaldehyd mit einem Carbonsäureanhydrid unter Bildung des entsprechenden Anhydrids, katalysiert durch ein Eisen(III)salz ausgewählt aus der Gruppe bestehend aus Eisenacetat, Eisensubcarbonat, Eisencarbonat, Eisenoxid, Eisenoxidhydrat, Eisenoxalat, Eisenlactat, Eisentartrat und Gemische dieser Eisen(III)salze,
- Umsetzung des gebildeten Anhydrids
(a) in Gegenwart von Alkoholen zu Zimtsäureester
oder
(b) mit Wasser zu Zimtsäure.

2. Verfahren nach Anspruch 1, **dadurch gekennzeichnet, dass** die Einsatzmenge an Eisen(III)salzen 0,0001-0,2 Moläquivalente bezogen auf die Einsatzmenge an Aldehyd beträgt.

3. Verfahren nach einem der Ansprüche 1 oder 2, **dadurch gekennzeichnet, dass** als Carbonsäureanhydrid Essigsäureanhydrid eingesetzt wird.

4. Verfahren nach einem der Ansprüche 1 bis 3, **dadurch gekennzeichnet, dass** das Carbonsäureanhydrid in Mengen von 1-5 Mol pro Mol Aldehyd eingesetzt wird.

5. Verfahren nach einem der Ansprüche 1 bis 4, **dadurch gekennzeichnet, dass** die Reaktion bei Temperaturen von 100-200 °C durchgeführt wird.

6. Verfahren nach einem der Ansprüche 1 bis 5, **dadurch gekennzeichnet, dass** die Reaktion unter Ausschluss von Sauerstoff, Wasser und Kohlendioxid durchgeführt wird.

7. Verfahren nach einem der Ansprüche 1 bis 6, **dadurch gekennzeichnet, dass** die Reaktion in Gegenwart eines Schutzgases durchgeführt wird.

8. Verfahren nach einem der Ansprüche 1 bis 7, **dadurch gekennzeichnet, dass** der gemäß Alternative (a) gebildete Ester durch saure oder alkalische Verseifung zu Zimtsäure umgesetzt wird.

9. Verfahren nach Anspruch 8, **dadurch gekennzeichnet, dass** Natronlauge oder Kalilauge eingesetzt werden.

10. Verfahren nach einem der Ansprüche 8 oder 9, **dadurch gekennzeichnet, dass** eine Lauge in einer Menge von 1-5 Mol pro Mol Ester eingesetzt wird.

## Claims

1. Process for the preparation of cinnamic acid esters and/or cinnamic acid, comprising the following steps:
- condensation of benzaldehyde or substituted benzaldehyde with a carboxylic anhydride to form the corresponding anhydride, catalysed by an iron(III) salt selected from the group consisting of iron acetate, iron subcarbonate, iron carbonate, iron oxide, iron oxide hydrate, iron oxalate, iron lactate, iron tartrate and mixtures of these iron(III) salts,
- reaction of the resulting anhydride
(a) in the presence of alcohols to form cinnamic acid esters
or
(b) with water to form cinnamic acid.

2. Process according to claim 1, **characterised in that** the amount of iron(III) salts used is from 0.0001 to 0.2 molar equivalent, based on the amount of aldehyde used.

3. Process according to either claim 1 or claim 2, **characterised in that** acetic anhydride is used as the carboxylic anhydride.

4. Process according to any one of claims 1 to 3, **characterised in that** the carboxylic anhydride is used in amounts of from 1 to 5 mol. per mol. of aldehyde.

5. Process according to any one of claims 1 to 4, **characterised in that** the reaction is carried out at temperatures of from 100 to 200°C.

6. Process according to any one of claims 1 to 5, **characterised in that** the reaction is carried out with the exclusion of oxygen, water and carbon dioxide.

7. Process according to any one of claims 1 to 6, **characterised in that** the reaction is carried out in the presence of a protecting gas.

8. Process according to any one of claims 1 to 7, **characterised in that** the ester formed according to alternative (a) is converted into cinnamic acid by acidic or alkaline saponification.

9. Process according to claim 8, **characterised in that** sodium hydroxide solution or potassium hydroxide solution is used.

10. Process according to either claim 8 or claim 9, **characterised in that** a lye is used in an amount of from 1 to 5 mol. per mol. of ester.

## Revendications

1. Procédé de production d'esters d'acide cinnamique et/ou de production d'acide cinnamique, comportant les étapes suivantes :
- condensation du benzaldéhyde ou du benzaldéhyde substitué avec un anhydride d'acide carboxylique en formant l'anhydride correspondant, catalysé par un sel de fer (III) choisi dans le groupe constitué de l'acétate de fer, du sous-carbonate de fer, du carbonate de fer, de l'oxyde de fer, de l'oxyde de fer hydraté, de l'oxalate de fer, du lactate de fer, du tartrate de fer et des mélanges de ces sels de fer (III),
- réaction de l'anhydride formé
(a) en présence d'alcools pour donner un ester d'acide cinnamique
ou
(b) avec de l'eau pour donner un acide cinnamique.

2. Procédé selon la revendication 1, **caractérisé en ce que** la quantité mise en oeuvre de sels de fer (III) est de 0,0001 à 0,2 équivalent molaire par rapport à la quantité mise en oeuvre d'aldéhyde.

3. Procédé selon l'une des revendications 1 ou 2, **caractérisé en ce que** l'on utilise comme anhydride d'acide carboxylique l'anhydride acétique.

4. Procédé selon l'une des revendications 1 à 3, **caractérisé en ce que** l'anhydride d'acide carboxylique est utilisé en des quantités de 1 à 5 moles par mole d'aldéhyde.

5. Procédé selon l'une des revendications 1 à 4, **caractérisé en ce que** la réaction est réalisée à des températures de 100 à 200°C.

6. Procédé selon l'une des revendications 1 à 5, **caractérisé en ce que** la réaction est réalisée sous exclusion d'oxygène, d'eau et de dioxyde de carbone.

7. Procédé selon l'une des revendications 1 à 6, **caractérisé en ce que** la réaction est réalisée en présence d'un gaz protecteur.

8. Procédé selon l'une des revendications 1 à 7, **caractérisé en ce que** l'ester formé d'après la variante (a) est converti en acide cinnamique par saponification acide ou alcaline.

9. Procédé selon la revendication 8, **caractérisé en ce que** l'on utilise de la soude caustique ou de la potasse caustique.

10. Procédé selon l'une des revendications 8 ou 9, **caractérisé en ce que** l'on utilise une lessive alcaline en une quantité de 1 à 5 moles par mole d'ester.
